(19) **Europäisches Patentamt**
**European Patent Office**
**Office européen des brevets**

(11) Veröffentlichungsnummer: **0 004 649**
**B1**

(12) # EUROPÄISCHE PATENTSCHRIFT

(45) Veröffentlichungstag der Patentschrift: **07.01.81**

(51) Int. Cl.³: **C 07 C 103/46**, G 01 N 33/48

(21) Anmeldenummer: **79100967.3**

(22) Anmeldetag: **30.03.79**

(54) **N-Carboxyacylaminosäureester, Verfahren zu ihrer Herstellung sowie ihre Verwendung.**

(30) Priorität: **11.04.78 DE 2815555**

(43) Veröffentlichungstag der Anmeldung: **17.10.79 Patentblatt 79/21**

(45) Bekanntmachung des Hinweises auf die Patenterteilung: **07.01.81 Patentblatt 81/1**

(84) Benannte Vertragsstaaten: **BE CH DE FR GB IT NL SE**

(56) Entgegenhaltungen:
**CHEMICAL ABSTRACTS, vol. tr, 1966, abstract 5764b. Columbus, Ohio, USA**
**B.F. ERLANGER et al. "The action of chymotrypsin on two new chromogenic substrates"**

(73) Patentinhaber: **BAYER AG, Zentralbereich Patente, Marken und Lizenzen, D-5090 Leverkusen 1, Bayerwerk (DE)**

(72) Erfinder: **Schnabel, Eugen, Dr., Schimmelweg 6, D-5600 Wuppertal 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 004 649 B1

ACTORUM AG.

# N-Carboxyacylaminosäureester, Verfahren zu ihrer Herstellung sowie ihre Verwendung

Die vorliegende Erfindung betrifft Carboxyacylaminosäureester, Verfahren zu ihrer Herstellung sowie ihre Verwendung als chromogene Substrate zur kontinuierlichen und diskontinuierlichen Bestimmung von Enzymen mit chymotrypsinartiger Spezifität, insbesondere von Cathepsin G.

Es ist bereits bekannt geworden, dass β-Naphthylester von Acetyl- bzw. Benzoyl-Phenylalanin für die Bestimmung der Chymotrypsine und insbesondere von Cathepsin G, dem chymotrypsinartigen Enzym aus Granulocyten verwendet werden können. [P.M. Starkey und A.J. Barrett, Biochem. J. 155, 273 (1976).] Dabei wird das freigesetzte β-Naphthol nach dem Kuppeln mit Diazoniumverbindungen colorimetrisch bestimmt. Nach H. Fritz, F. Woitinas und E. Werle, H.S.Z. Physiol. Chem. 345, 168 (1966) sind die dabei für Chymotrypsin in stark eiweisshaltigen Lösungen erhaltenen Werte nicht reproduzierbar. Zudem sind beide Substrate sehr lipophil und auch durch Zusatz von Detergentien können kaum homogene Lösungen erhalten werden. Die Verwendung von in Wasser leichter löslichen Substraten, wie Succinyl-L-phenylalanin-p-nitroanilid [W. Nagel, F. Willig, W. Peschke und F.H. Schmidt, H.S.Z. Physiol. Chem. 340, 1 (1965)], Glutaryl-L-phenylalanin-p-nitroanilid [B.F. Erlanger, F. Edel und A.G. Cooper, Arch. Biochem. Biophys. 115, 206 (1966)] oder Glutaryl-diglycyl-L-phenylalanin-β-naphthylamid [H. Rinderknecht und R.M. Fleming, Clin. Chim. Acta 59, 139 (1975)] ist nur bei der Bestimmung von Chymotrypsin möglich, weil diese Substrate von Cathepsin G nicht gespalten werden [P.M. Starkey und A.J. Barrett, Biochem. J. 155, 273 (1976)]. Es wurde nun gefunden, dass die N-Carboxyacyl-L-aminosäureester der Formel I

$$\begin{array}{l} \text{HOOC-X-CONH-}\overset{}{\text{CH}}\text{-COOY} \\ \qquad\qquad\qquad\quad | \\ \qquad\qquad\qquad\quad \text{CH}_2 \\ \qquad\qquad\qquad\quad | \\ \qquad\qquad\qquad\quad \text{Z} \end{array} \qquad \text{(I)}$$

worin

X gegebenenfalls durch $C_1$–$C_4$-Alkyl-substituiertes, geradkettiges $C_2$–$C_4$-Alkylen oder Alkenylen oder gegebenenfalls substituiertes o-Phenylen,

Y gegebenenfalls substituiertes Phenyl oder Naphthyl und

Z gegebenenfalls durch Hydroxy substituiertes Phenyl oder Indolyl sind

gut in wässrigen Systemen löslich sind und sich hervorragend als Substrate für die Chymotrypsine und besonders auch für Cathepsin G eignen.

Geeignete Reste X sind beispielsweise Äthylen, Propylen, Butylen oder o-Phenylen; geeignete Reste Y sind beispielsweise α-Naphthyl, β-Naphthyl, Phenyl und 4-Nitrophenyl; geeignete Reste Z sind beispielsweise Phenyl, 4-Hydroxyphenyl und β-Indolyl.

Die Aminosäurederivate gehören vorzugsweise der L-Reihe an, doch können sie auch racemisch vorliegen.

Bevorzugte Verbindungen der Formel (I) sind Succinyl-L-phenylalanin-β-naphthylester, Succinyl-L-phenylalanin-4-nitrophenyl-ester, Adipinyl-L-phenylalanin-β-naphthylester und Adipinyl-L-phenylalanin-4-nitrophenyl-ester. Die Aktivität dieser Enzyme kann nicht nur, wie bei der Verwendung von Acetyl bzw. Benzoyl-phenylalanin-β-naphthylester beschrieben, nach dem Abstoppen der Enzymreaktion und der Kupplung des gebildeten β-Naphtols mit Diazoniumverbindungen in einem Endpunktsverfahren bestimmt werden, sondern erfindungsgemäss auch kontinuierlich anhand der durch die Freisetzung der Phenole und Naphthole bedingten Extinktionszunahme bei einer Wellenlänge, bei der die Ester praktisch nicht absorbieren. Wie ein Vergleich der Absorptionsspektren von Succinyl-L-phenylalanin-β-naphthylester und von β-Naphthol in Abb. 1 zeigt, verschiebt sich das Absorptionsmaximum bei der Spaltung unter Intensivierung nach längeren Wellenlängen, und die molare Extinktion von β-Naphthol beträgt in einem 0,25 m Tris-(hydroxymethyl-) aminomethan-Puffer pH 7,2 bei 329 nm 1790 $Mol^{-1} \cdot cm^{-1}$. Mit diesem Substrat ist eine genaue Bestimmung der Chymotrypsine und von Cathepsin G auch in stark eiweisshaltigen Lösungen möglich. Die Empfindlichkeit des Testes kann dadurch gesteigert werden, dass man, wie für das Endpunktsverfahren beschrieben [H.A. Ravin, P. Bernstein und A.M. Seligman, J. biol. Chem. 208, 1 (1954)], das β-Naphthol nach einer vorgegebenen Zeit mit einer Diazoniumverbindung kuppelt und die sehr viel höhere Extinktion des Azoderivates bestimmt. Noch grösser ist die Differenz der Extinktionsmaxima bei Succinyl-L-phenylalanin-p-nitrophenylester ($\lambda_{max.}$ 270 nm) und p-Nitrophenol ($\lambda_{max.}$ 400 nm). Mit Succinyl-L-phenylalanin-β-naphthylester als Substrat werden die Messungen bei pH-Werten zwischen pH 6,5 und 8,5 durchgeführt; unterhalb pH 6,5 ist das Substrat in wässrigen Systemen schwer löslich, oberhalb von pH 8,5 verläuft die Spontanhydrolyse der aktivierten Ester so schnell, das die Bestimmungen ungenau werden. Die Testlösungen können gegebenenfalls Zusätze wie Detergentien und/oder organische Lösungsmittel enthalten. Geeignete Detergentien sind z.B. Polyäthylenglykol-fettalkoholäther, beispielsweise Polyäthylenglykol mit etwa 20 Äthylenoxideinheiten, veräthert mit Laurylalkohol oder Derivate der Cholsäuren. Als organische Lösungsmittel eignen sich beispielsweise Dimethylsulfoxid, Dimethylformamid, Dimethylacetamid oder Hexamethylphosphorsäuretriamid.

Die erfindungsgemässen Substrate sind sehr spezifisch für die Chymotrypsine und für Cathepsin G. In der Tabelle 1 ist die Spaltbarkeit von Succinyl-L-phenylalanin-β-naphthylester durch verschiedene Proteasen, bezogen auf α-Chymotrypsin (100%), angegeben.

Für α-Chymotrypsin bzw. Cathepsin G und Succinyl-L-phenylalanin-β-naphthylester wurden die Michaeliskonstanten in dem obigen 0,25 m Tris-(hydroxymethyl)-aminomethan/Salzsäure-Puffer pH 7,2 bei 25°C nach H. Lineweaver und D. Burk [J. Amer. Chem. Soc. 56, 658 (1934)] zu $4{,}2\cdot10^{-4}$ Mol/l bzw. $4{,}5\cdot10^{-4}$ Mol/l bestimmt. Die entsprechenden Werte für das Substrat Adipinyl-L-phenylalanin-β-naphthylester wurden zu $6{,}4\cdot10^{-4}$ Mol/l bzw. $7{,}2\cdot10^{-4}$ Mol/l gefunden.

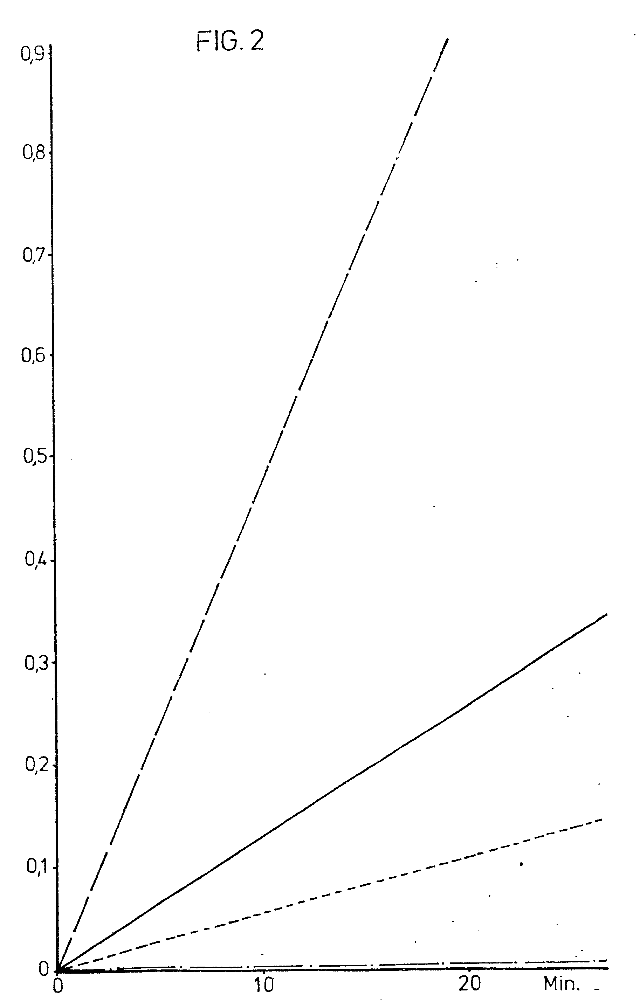

In Abb. 2 sind die bei der Spaltung des erfindungsgemässen Succinyl-L-phenylalanin-β-naphthylesters bzw. des bekannten Glutaryl-L-Phenylalanin-p-nitroanilids resultierenden Extinktionszunahmen aufgezeichnet. Berücksichtigt man, dass der molare Extinktionskoeffizient von p-Nitroanilin mit 8800 $Mol^{-1}\cdot cm^{-1}$ fast um den Faktor 5 grösser ist als der von β-Naphthol, so ergibt sich für das erfindungsgemässe Substrat eine mindestens um den Faktor 1000 raschere Spaltung. Die Nachweisgrenze für Chymotrypsin wird dadurch mindestens um den Faktor 100 erhöht. In Anbetracht der hohen Spezifität der erfindungsgemässen Verbindungen als Substrate für Chymotrypsin und chymotrypsinartige Enzyme (vgl. Tabelle 1) stellen diese Derivate eine Bereicherung für die Enzymchemie insbesondere für die Bestimmung der Enzyme dar.

Die erfindungsgemässen Substrate sind im Hinblick auf die pathogenetische Bedeutung der neutrophilen Proteasen und insbesondere von Cathepsin G [A. Janoff, Ann. Rev. Med. 23, 177 (1972)] wertvolle diagnostische Hilfsmittel.

Die erfindungsgemässen N-Carboxyacyl-aminosäureester werden erhalten, indem man die Aminosäureester der allgemeinen Formel II

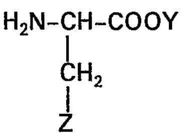

(II)

in der Z und Y die obengenannte Bedeutung haben, bei pH-Werten zwischen 2 und 7, vorzugsweise bei pH 4–6, in geeigneten Lösungsmitteln mit Dicarbonsäureanhydriden der allgemeinen Formel III

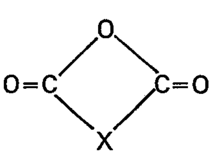

(III)

worin X die oben genannte Bedeutung hat, umsetzt.

Als geeignete Lösungsmittel für diese Umsetzung seien insbesondere Aceton, Acetonitril, Chloralkane, Dioxan, Dimethylformamid, Eisessig, Tetrahydrofuran und Wasser genannt. Sie können auch in Mischungen verwendet werden. Den pH-Wert der Reaktionslösungen hält man entweder durch Zugabe von Basen oder mit Pufferlösungen im gewünschten Bereich; unter Umständen kann man die Esterhydrochloride ohne Basenzusatz für die Umsetzungen einsetzen.

Die Reaktionen werden bei Temperaturen zwischen −10°C und +50°C durchgeführt, vorzugsweise bei 0–10°C, wobei man die Reaktionslösungen zunächst kühlt und sich dann erwärmen lässt.

Als geeignete cyclische Dicarbonsäureanhydride seien insbesondere die Anhydride der Bernsteinsäure und der Phthalsäure genannt. Sie können gegebenenfalls noch zusätzliche Substituenten tragen.

Es ist zwar bereits bekannt geworden, dass die cyclischen Anhydride der Dicarbonsäuren mit Aminosäuren und ihren Derivaten, wie den freien Alkylestern [C. Berse, L. Piché, L. Lachance und G. Laflamme, J. org. Chem. 27, 3489 (1962); B. Helferich und W. Wesemann, Chem. Ber. 100, 421 (1967)] oder gegebenenfalls substituierten Amiden, insbesondere den p-Nitroaniliden [B.F. Erlanger, F. Edel und A.G. Cooper, Arch. Biochem. Biophys. 115, 206 (1966)] unter vorzugsweise alkalischen Bedingungen reagieren. Phenyl- bzw. naphthyl-Ester der Aminosäuren als sogenannte «aktivierte Ester» [G. Wendlberger in E. Müller (Hrsg.), Houben-Weyl, Methoden der Organischen Chemie, 4. Auflage 1974, Bd. 15/2, Georg Thieme Verlag, Stuttgart] sind bisher noch nicht mit cyclischen Dicarbonsäureanhydriden umgesetzt worden. Es war anzunehmen, dass die aktivierten Aminosäureester unter den üblichen Reaktionsbedingungen bei den Umsetzungen mit den Dicarbonsäureanhydriden durch Reaktion mit der eigenen Aminogruppe oder in Gegenwart von Wasser durch Hydrolyse gespalten würden. Überraschend wurde nun gefunden, dass die erfindungsgemässen N-Carboxyacylverbindungen bei den Umsetzungen mit den Hydrochloriden der aktivierten Aminosäureester im sauren Milieu in hohen Ausbeuten erhalten werden.

Alternativ können die erfindungsgemässen N-Carboxyacylaminosäureester aber auch durch acidolytische Spaltung der ω-tert. Butylester der N-Carboxyacylaminosäureester der Formel (IV)

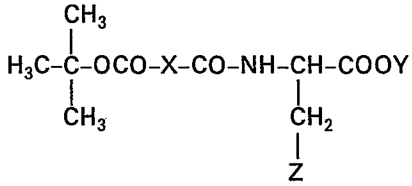

(IV)

worin X, Y und Z die obengenannte Bedeutung haben,

erhalten werden.

Für die Acidolyse geeignete Säuren sind beispielsweise Mineralsäuren oder ihre Lösungen in vorzugsweise wasserfreien organischen Lösungsmitteln, wie Äther, Äthylacetat, Tetrahydrofuran, Dioxan, Eisessig oder Methanol. Die acidolytischen Spaltungsreaktionen werden bei Temperaturen zwischen −10°C und +50°C, vorzugsweise bei 0–25°C und gegebenenfalls unter Stickstoff durchgeführt.

Die für die Acidolyse erforderlichen Ausgangsverbindungen (IV) werden durch Umsetzen aktivierter Derivate von Mono-tert.butylestern der entsprechenden Dicarbonsäuren mit Aminosäuren, die gegebenenfalls silyliert sein können oder Aminosäurealkylestern und anschliessende Reaktion mit den Phenolen bzw. Naphtholen nach dem Freilegen der Carboxylgruppe der Aminosäuren und entsprechender Aktivierung erhalten.

Ausserdem werden die Verbindungen der Formel (IV) erhalten, indem man monoaktivierte Derivate der Dicarbonsäuren, deren zweite Carboxylgruppe frei ist, mit Aminosäureestern zu den N-Carboxyacylaminosäureestern reagieren lässt und diese nach dem Schutz der ω-Carboxylgruppe und nach der Deblockierung der Aminosäurecarboxylgruppe, wie oben beschrieben, in die Mono-tert.butylester-carboxyacylaminosäure-Phenylester bzw. -Naphthylester überführt.

Beispiel 1

Succinyl-L-phenylalanin-β-naphthylester

a) tert.Butoxycarbonyl-L-phenylalanin-β-naphthylester.

Zu einer Lösung von 7,8 g tert.-Butoxycarbonyl-L-Phenylalanin und 5,3 g β-Naphthol in 50 ml absolutem Tetrahydrofuran fügt man bei 0 °C 6,2 g Dicyclohexylcarbodiimid und hielt das Reaktionsgemisch 12 Stunden bei 4 °C. Dann wurde der ausgefallene Dicyclohexylharnstoff durch Absaugen abgetrennt und das Filtrat im Vakuum zur Trockne eingedampft. Der kristalline Rückstand wurde zweimal aus Essigester/Cyclohexan und schliesslich aus Acetonitril umkristallisiert. Man erhielt 8,5 g (73% der Theorie) vom Schmelzpunkt 118–119 °C.

b) L-Phenylalanin-β-naphthylester-hydrochlorid

8,6 g der (nach 1a) dargestellten Verbindung wurden in 75 ml absolutem Dioxan gelöst. Zu dieser Lösung fügt man 100 ml 5 n ätherische HCl und 5 ml Trifluoressigsäure. Man rührte die Mischung 3 Stunden bei 20 °C und fällte das Hydrochlorid schliesslich durch Zugabe von 100 ml Diisopropyläther und 100 ml Cyclohexan zur Reaktionslösung quantitativ aus. Beim Abfiltrieren wurden 6,8 g (94% der Theorie) farblose Kristalle erhalten vom Schmelzpunkt 219–221 °C; nach dem Umkristallisieren aus Acetonitril: 221–222 °C.

c) Succinyl-L-phenylalanin-β-naphthylester

Zu einer Suspension von 3,3 g der nach 1b) erhaltenen Verbindung und 1 g Bernsteinsäureanhydrid in 50 ml Acetonitril fügte man bei 4 °C 10 ml Wasser und schliesslich unter weiterem Kühlen und Rühren 4 n Natronlauge bis zum Erreichen von pH 5,0. Unter Aufrechterhaltung dieses pH-Wertes wurde nach 20 Minuten 2 g Bernsteinsäureanhydrid portionsweise im Abstand von je 10 Minuten unter Rühren eingetragen. 1 Stunde nach der letzten Zugabe wurde die Reaktionslösung im Vakuum auf ein Volumen von ca. 20 ml eingeengt und dann mit 150 ml Essigester versetzt. Man extrahierte die Lösung anschliessend mit 20 ml 2 n Schwefelsäure und weitere fünf Male mit jeweils 50 ml warmem und schliesslich mit 50 ml heissem Wasser. Dann wurde der Essigester im Vakuum abdestilliert und der kristalline Rückstand dreimal aus Acetonitril umkristallisiert. Ausbeute 3,44 g (88% der Theorie) vom Schmelzpunkt 146–147 °C.

Beispiel 2

Adipyl-L-phenylalanin-β-naphthylester

a) Adipinsäure-mono-p-nitrophenylester

Zu 25,6 g Adipinsäureanhydrid fügte man 28 g fein pulversisiertes p-Nitrophenol und rührte das Gemisch über Nacht bei Raumtemperatur, wobei der Kolbeninhalt erstarrte. Die Masse wurde unter Rühren geschmolzen und die Schmelze in 350 ml Essigester gelöst. Man extrahierte die Lösung mehrmals mit Wasser und destillierte den Essigester im Vakuum ab. Der Rückstand wurde dreimal aus Acetonitril umkristallisiert. Ausbeute: 45,2 g (85% der Theorie) mit Schmelzpunkt 104–105 °C.

b) Adipyl-L-phenylalanin-benzylester

16,5 N-Trimethylsiliyl-L-phenylalanin-benzylester und 13,5 g Adipinsäure-mono-p-nitrophenylester wurden in 30 ml absolutem Dioxan gelöst und diese Lösung 24 Stunden bei Raumtemperatur gehalten. Man erwärmte die Mischung dann 5 Stunden auf ca. 60 °C und destillierte das Dioxan im Vakuum ab. Den Rückstand löste man in 150 ml Essigester und extrahierte die Lösung mehrmals mit je 30 ml 2 n Schwefelsäure und schliesslich mit Wasser. Nach dem Abdestillieren des Essigesters in Vakuum wurde ein gelblicher Sirup erhalten. Zum Abtrennen des p-Nitrophenols wurde über eine zu ¾ mit neutralem und zu ¼ mit basischem Aluminiumoxid gefüllte Säule mit Cyclohexan als Lösungsmittel chromatographiert. Beim Einengen der die Substanz enthaltenden farblosen Eluate erhielt man 17,8 g (93% der Theorie) sirupöse Substanz mit RF-Wert von 0,90 in Chloroform:Methanol:Essigsäure 190:10:6 (Vol).

c) N-tert.Butyl-adipyl-L-phenylalaninbenzylester

Zu einer Lösung von 10 g der nach 2b) erhaltenen Substanz in 50 ml Methylenchlorid fügte man 0,1 ml konzentrierte Schwefelsäure und 75 ml Isobutylen. Man hielt die Reaktionsmischung unter gelegentlichem Schütteln 4 Tage im verschlossenen Gefäss bei 20 °C.

Dann setzte man 1 ml Triäthylamin zu der Reaktionsmischung und extrahierte sie anschliessend mehrfach mit einer 5prozentigen Natriumbikarbonatlösung und schliesslich mit Wasser. Nach dem Abdestillieren des Isobutylens und des Methylenchlorids wurde der sirupöse Rückstand in 25 ml Diisopropyläther gelöst und unter Verwendung von Diisopropyläther, wie bei 2b) beschrieben, über eine mit neutralem bzw. im oberen Viertel mit basischem Aluminiumoxid gefüllte Säule chromatographiert. Die die gewünschte Substanz enthaltenden Eluate wurden vereinigt und die Lösungsmittel im Vakuum abdestilliert. Man erhielt so 10,2 g (86% der Theorie) farblosen Sirup; $[a]^{20}_{D}$ −9,3° (c 1, in Dimthylformamid).

d) tert.Butyl-adipyl-L-phenylalanin

Durch eine Lösung von 8,8 g nach 2c) dargestellter Substanz in 100 ml absolutem Äthanol liess man nach Zusatz von aus 1 g Palladiumchlorid frisch bereitetem Palladium-Mohr unter Rühren einen langsamen Wasserstoffstrom hindurchperlen. Nach 6 Stunden filtrierte man die Lösung zum Abtrennen des Katalysators und engte das Filtrat im Vakuum ein. Dabei kristallisierten 7,2 g (100% der Theorie) vom Schmelzpunkt 90 °C aus.

e) tert.Butyl-adipyl-L-phenylalanin-β-naphthylester

Zu einer Lösung von 4,5 g der nach 2d) erhaltenen Substanz und 1,8 g β-Naphthol in 50 ml absolutem Tetrahydrofuran fügte man bei 0 °C 2,6 g Dicyclohexylcarbodiimid und hielt die Reaktionsmischung 15 Stunden bei 4 °C. Man trennte den Dicyclohexylharnstoff durch Abfiltrieren ab und verdampfte das Tetrahydrofuran im Vakuum. Der dabei erhaltene Sirup wurde in wenig Essigester gelöst und über neutrales Aluminiumoxid chromatographiert,

indem man die Säule zunächst mit Äther-Petroläther 1:1, Äther, Methylenchlorid und Methanol eluierte. Beim Einengen der vereinigten Äther- und Methylenchlorid-Eluate erhielt man 1,8 g (30% der Theorie) eines unter Äther kristallisierenden Sirups vom Schmelzpunkt 80 °C.

f) Adipyl-L-phenylalanin-β-naphthylester

1,7 g des unter 2e) beschriebenen tert.Butylesters wurden mit 10 ml wasserfreier Trifluoressigsäure übergossen. Nach einstündigem Stehen bei 20 °C unter Stickstoff wurde die Lösung in Vakuum eingeengt und der sirupöse Rückstand mit Äther versetzt. Man destillierte den Äther im Vakuum ab und wiederholte dieses Verfahren mehrere Male. Beim Stehen unter wenig Acetonitril kristallisierten 1,39 g (95% der Theorie) farblose Substanz, die abgesaugt und gut mit Äther gewaschen wurde; Schmelzpunkt 94 °C.

Beispiel 3

Succinyl-L-phenylalanin-p-nitrophenylester

a) L-Phenylalanin-p-nitrophenylester-hydrochlorid

Zu einer Lösung von 21 g tert.Butyloxycarbonyl-L-phenyl-alaninin-p-nitrophenylester in 50 ml wasserfreiem Tetrahydrofuran fügte man 250 ml 5 n ätherischen Chlorwaserstoff und rührte die Reaktionsmischung 1 Stunde unter Feuchtigkeitsausschluss bei 22 °C. Man setzte 250 ml absoluten Äther zu der entstandenen Fällung und isolierte die gelblichen Kristalle durch Absaugen: 15,9 g (99% der Theorie) vom Schmelzpunkt 191–193 °C.

b) Succinyl-L-phenylalanin-p-nitrophenylester

8 g (25 mMol) nach 3a) erhaltene Substanz wurden unter Erwärmen in 150 ml Acetonitril gelöst und mit 2,5 g Bernsteinsäureanhydrid versetzt. Nach 30 Minuten fügte man bei 4 °C 20 ml Wasser zu der Mischung und hielt den pH-Wert der Reaktionslösung durch Zugabe von 8 n Natronlauge auf 5,5. Man versetzte mit weiteren 2,5 g Bernsteinsäureanhydrid und rührte das Gemisch weitere 1,5 Stunden bei pH 5,5. Dann wurden die ausgefallenen Kristalle durch Absaugen isoliert; eine weitere Fraktion wurde beim Einengen des Filtrates erhalten. Die vereinigten Präparationen wurden mehrmals aus Acetonitril umkristallisiert; 6,8 g (67% der Theorie) vom Schmelzpunkt 168 °C.

Beispiel 4

Bestimmung von Enzymen mit chymotrypsinartiger Spezifität unter Verwendung von Succinyl-L-phenylalanin-β-naphthylester

A: Herstellung der Lösungen

1) 0,25 m Trispuffer: 30,29 g Tris-(hydroxymethyl) aminomethan und 0,203 g Magnesiumchlorid-hexahydrat p.A. sowie 0,5 g Polyäthylenglykol mit etwa 20 Äthylenoxideinheiten veräthert mit Laurylalkohol werden in einem 1-Liter Messkolben in 700 ml Wasser gelöst und 1 n Salzsäure bis pH 7,2 zugefügt. Man füllt den Messkolben dann bis zur Marke.

2) Substratlösung: 39,14 mg Succinyl-L-phenylalanin-β-naphthylester werden in 1 ml Dimethylsulfoxid gelöst. Diese Lösung ist im Eisschrank praktisch unbegrenzt haltbar.

B: Durchführung des Tests

1) Nullwert (Bestimmung der Spontanhydrolyse): Zu 2,5 ml des obigen Trispuffer (A1) fügt man 0,025 ml Substratlösung (A2) und misst die Extinktionszunahme bei 329 nm gegen eine Vergleichslösung, die anstelle der Substratlösung das gleiche Volumen Dimethylsulfoxid enthält. Man bestimmt so $\Delta_{OD\ Null}$

2) Testprobe: Man mischt Trispuffer und Enzymlösung (0,01–0,5 ml) so, dass man ein Volumen von 2,5 ml erhält. Nach dem Homogenisieren fügt man 0,025 ml Substratlösung zu dem Ansatz und misst den Extinktionsanstieg bei 329 nm gegen eine Vergleichsküvette, die die gleiche Menge Testlösung enthält und 0,025 ml Dimethylsulfoxid anstelle der Substratlösung. Man erhält

$\Delta_{OD\ Test}$

3) Berechnung der Enzymaktivität: Aus den Werten der Extinktionszunahme in der Testlösung und im Vergleichs- oder Leerwert errechnet sich die enzymatische Aktivität nach folgender Formel:

$$\text{Aktivität (nkat)} = \frac{\Delta_{OD\ Test} - \Delta_{OD\ Null}}{1 \cdot t \cdot 0{,}042}$$

In dieser Formel beziehen sich die $\Delta_{OD}$-Werte auf den Extinktionsanstieg in der Zeit t (in Minuten) bei der Schichtdicke 1 (in cm).

**Patentansprüche**

1. N-Carboxyacylaminosäureester der Formel

```
HOOC-X-CONH-CH-COOY
            |
            CH2
            |
            Z
```

worin
X gegebenenfalls durch $C_1$–$C_4$-Alkyl-substituiertes, geradkettiges $C_2$–$C_4$-Alkylen oder Alkenylen oder gegebenenfalls substituiertes o-Phenylen,
Y gegebenenfalls substituiertes Phenyl oder Naphthyl und
Z gegebenenfalls durch Hydroxy substituiertes Phenyl oder Indolyl sind.

2. Verbindungen nach Anspruch 1 in der L-Form.

3. Succinyl-L-phenylalanin-β-naphthylester.

4. Succinyl-L-phenylalanin-(4-nitrophenyl)-ester.

5. Adipinyl-L-phenylalanin-β-naphthylester.

6. Adipinyl-L-phenylalanin-(4-nitrophenyl)-ester.

7. Verfahren zur Herstellung der Verbindungen gemäss Anspruch 1, dadurch gekennzeichnet, dass man Aminosäureester der allgemeinen Formel

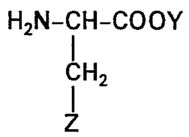

worin Y und Z die in Anspruch 1 genannte Bedeutung besitzen,
mit Dicarbonsäureanhydriden der Formel

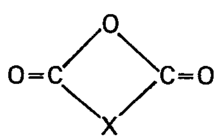

worin X die in Anspruch 1 genannte Bedeutung hat, umsetzt.

8. Verfahren zur Herstellung der Verbindungen gemäss Anspruch 1, dadurch gekennzeichnet, das man tert.-Butylester der Formel

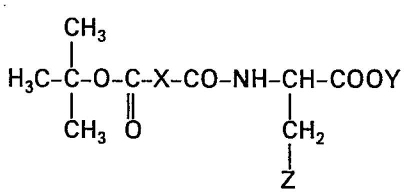

worin X, Y und Z die in Anspruch 1 genannte Bedeutung haben,
acidolytisch spaltet.

9. Verwendung der N-Carboxyacylaminosäureester gemäss Anspruch 1 als chromogene Substrate zur Bestimmung von Enzymen mit chymotrypsinartiger Spezifität.

10. Diagnostische Hilfsmittel enthaltend N-Carboxyacylaminosäureester gemäss Anspruch 1.

**Patent Claims**

1. N-carboxyacylaminoacid esters of the formula

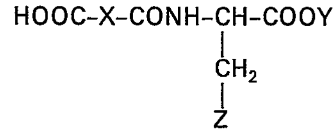

in which
X denotes straight-chain $C_2$ to $C_4$ alkylene or alkenylene which are optionally substituted by $C_1$ to $C_4$ alkyl, or optionally substituted o-phenylene,
Y denotes optionally substituted phenyl or naphthyl
and Z denotes phenyl or indolyl optionally substituted by hydroxyl.

2. Compounds according to claim 1, in the L-form.

3. Succinyl-L-phenylalanine β-naphthyl ester.

4. Succinyl-L-phenylalanine 4-nitrophenyl ester.

5. Adipyl-L-phenylalanine β-naphthyl ester.

6. Adipyl-L-phenylalanine 4-nitrophenyl ester.

7. A process for the production of the compounds according to claim 1, characterised in that amino acid esters of the general formula

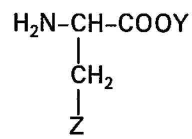

in which
Y and Z have the meaning mentioned in claim 1, are reacted with dicarboxylic acid anhydrides of the formula

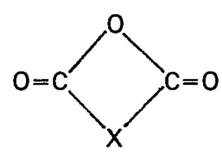

in which
X has the meaning mentioned in claim 1.

8. A process for the production of the compounds according to claim 1, characterised in that tert.-butyl esters of the formula

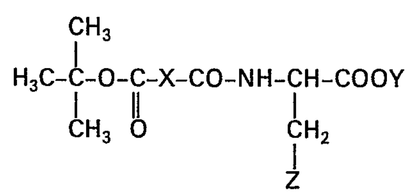

in which X, Y and Z have the meaning mentioned in claim 1, are split acidolytically.

9. Use of the N-carboxyacylaminoacid esters according to claim 1 as chromogenic substrates for the determination of enzymes with chymotrypsin-like specificity.

10. Diagnostic auxiliaries containing N-carboxyacylaminoacid esters according to claim 1.

## Revendications

1. Esters d'acides N-carboxyacylaminés répondant à la formule:

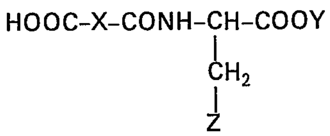

dans laquelle X représente un radical alkylène ou alcénylène à chaîne droite en $C_2$-$C_4$ éventuellement substitué par des groupes alkyle en $C_1$-$C_4$, ou un radical o-phénylène éventuellement substitué, Y représente un radical phényle ou naphtyle éventuellement substitué, et Z représente un radical phényle ou indolyle éventuellement substitué par des groupes hydroxy.

2. Composés selon la revendication 1, sous la forme L.

3. Ester β-naphtylique de la succinyl-L-phénylalanine.

4. Ester 4-nitrophénylique de la succinyl-L-phénylalanine.

5. Ester β-naphtylique de l'adipinyl-L-phénylalanine.

6. Ester 4-nitrophénylique de l'adipinyl-L-phénylalanine.

7. Procédé de préparation des composés selon la revendication 1, caractérisé en ce que l'on fait réagir des esters d'aminoacides répondant à la formule générale:

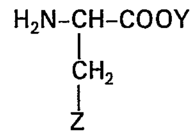

dans laquelle Y et Z ont les significations indiquées dans la revendication 1, avec des anhydrides d'acides dicarboxyliques répondant à la formule:

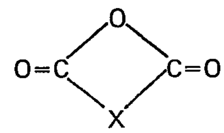

dans laquelle X a les significations indiquées dans la revendication 1.

8. Procédé de préparation des composés selon la revendication 1, caractérisé en ce que l'on soumet à scission par acidolyse des esters tert.-butyliques répondant à la formule:

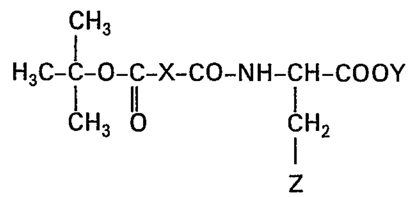

dans laquelle X, Y et Z ont les significations indiquées dans la revendication 1.

9. Utilisation des esters d'acides N-carboxyacylaminés selon la revendication 1, en tant que substrats chromogènes pour la détermination des enzymes à spécificité du type chymotrypsine.

10. Produits auxiliaires de diagnostic contenant des esters d'acides N-carboxyacylaminés selon la revendication 1.

Tabelle 1:
Relative Spaltungsraten von Succinyl-L-phenylalanin-β-naphthylester für verschiedene Proteasen, gemessen in einem 0,05% Polyäthylenglykol mit 20 Äthylenoxideinheiten, veräthert mit Laurylalkohol enthaltenden 0,25 m Tris-(hydroxymethyl)-aminomethan-Salzsäure-Puffer, pH 7,2

| Enzym | Einsatz/Test | Bedingungen | rel. Spaltungsrate (%) |
|---|---|---|---|
| α-Chymotrypsin | 8,5 NF-E+ | pH 7,2 | 100 |
| Cathepsin $D_2$ | 1 E[1] | pH 3,0 | 0 |
| Cathepsin G | 0,50 nkat[2] | pH 7,2 | 124 |
| Elastase (Granulozyten) | 0,25 nkat[3] | pH 7,2 | 0,5 |
| Elastase (Pankreas) | 2,0 nkat[3] | pH 7,2 | 0,4 |
| Kallikrein (Pankreas) | 4 E | pH 7,2 | 0 |
| Kallikrein (Plasma) | 0,1 nkat[4] | pH 7,2 | 0 |
| Plasmin | 0,2 CU[5] | pH 7,2 | 0,4 |
| Thrombin | 0,05 NIH-E | pH 7,2 | 0 |
| Trypsin | 11,5 NF-E | pH 7,2 | 2,0 |

+E = Einheiten; nkat = Enzyme Nomenclature, Recommendations (1972) of the International Union of Pure and Applied Chemistry and the International Union of Biochemistry, 1973, Elsevier, Amsterdam-New York, S. 26-27

1) bestimmt mit Hämoglobin als Substrat nach A.J. Barrett in J.T. Dingle (Hrsg.) Lysosomes, Laboratory Handbook, North Holland Publishing Co., Amsterdam-London 1972, S. 123
2) bestimmt mit Succinyl-L-phenylalanin-β-naphthylester als Substrat
3) bestimmt mit Succinyl-L-alanyl-L-alanyl-L-alanin-p-nitroanilid nach J. Bieth, B. Spiess, C.G. Wermuth, Biochem. Med. 11, 350 (1974)
4) bestimmt mit Benzoyl-L-prolyl-L-phenylalanyl-L-arginin-p-nitroanilid nach L.G. Svendsen, Deutsche Offenlegungsschrift 25 27 932
5) J.T. Sgouris, Vox. Sang. 5, 357 (1960)

Für α-Chymotrypsin bzw. Cathepsin G und Succinyl-L-phenylalanin-β-naphthylester wurden die Michaeliskonstanten in dem obigen 0,25 m Tris-(hydroxymethyl)-aminomethan/Salzsäure-Puffer pH 7,2 bei 25°C nach H. Lineweaver und D. Burk [J. Amer. Chem. Soc. 56, 658 (1934)] zu $4{,}2 \cdot 10^{-4}$ Mol bzw. $4{,}5 \cdot 10^{-4}$ bestimmt. Die entsprechenden Werte für das Substrat Adipinyl-L-phenylalanin-β-naphthylester wurden zu $6{,}4 \cdot 10^{-4}$ Mol gefunden.

Beschreibung der Abbildungen

Abb. 1: UV-Absorptionsspektrum von

_ _ _ _ _ _ Succinyl-L-phenylalanin-β-naphthylester $10^{-3}$ in 0,25 m Trispuffer pH 7,2 ($E_{315} = 270$; $E_{302} = 325$)

——— β-Naphthol $3{,}1 \cdot 10^{-4}$ m in 0,25 m Trispuffer pH 7,2 ($E_{329} = 1790$)

Abb. 2: Spaltung von Succinyl-L-phenylalanin-β-naphthylester und Glutaryl-L-phenylalanin-p-nitroanilid durch Chymotrypsin

_ _ _ _ _ _ $10^{-3}$ m Lösung von Succinyl-L-phenylalanin-β-naphthylester (2,5 ml) mit $8 \cdot 10^{-4}$ mg α-Chymotrypsin

. . . . . . . . Spontanhydrolyse von Succinyl-L-phenylalanin-β-naphthylester

——— $10^{-3}$ m Lösung von Glutaryl-L-phenylalanin-p-nitroanilid (2,5 ml) mit $8 \cdot 10^{-2}$ mg α-Chymotrypsin

- . - . - . $10^{-3}$ m Lösung von Glutaryl-L-phenylalanin-p-nitroanilid (2,5 ml) mit $8 \cdot 10^{-4}$ mg α-Chymotrypsin

FIG. 1

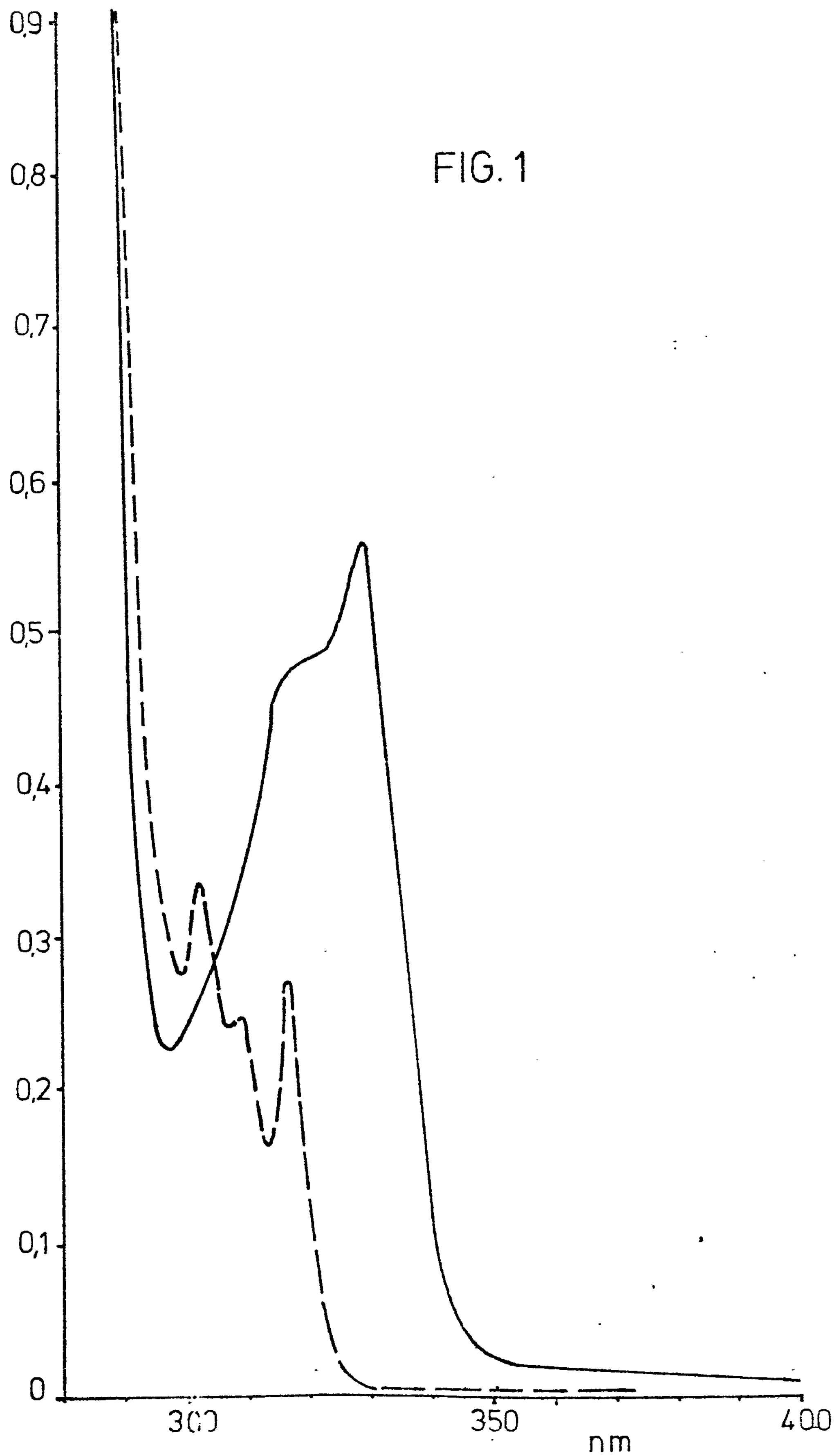

FIG. 2

2/2